# EUROPEAN PATENT SPECIFICATION

(11) **EP 0 948 332 B1**
(45) Date of publication and mention of the grant of the patent: **03.09.2003**
(21) Application number: 97948849.1
(22) Date of filing: 31.10.1997
(51) Int. Cl.: A61K 31/52, A61K 9/06, A61K 47/00, A61P 31/22

(54) **ANTIHERPETIC MEDICATED STICK CONTAINING ACYCLOVIR FOR TOPICAL APPLICATION**
ACYCLOVIRHALTIGE ANTIHERPESARZNEISTIFT ZUR TOPISCHEN ANWENDUNG
BATONNET MEDICAMENTEUX ANTIHERPETIQUE CONTENANT DE L'ACYCLOVIR POUR L'APPLICATION TOPIQUE

(30) Priority: 31.10.1996 IT MI962258
(43) Date of publication of application: 13.10.1999
(73) Proprietor: RECORDATI S.A., 6830 Chiasso (CH)
(72) Inventor: SANTUS, Giancarlo, I-20146 Milan (IT); GOLZI, Roberto, I-26013 Cremona (IT); GARAVAGLIA, Antonio, I-20141 Milan (IT)
(74) Representative: Gervasi, Gemma, Dr.
(86) International application number: EP9706022
(87) International publication number: WO98018472

(56) References cited:
- WO-A-93/00905
- WO-A-96/24355

## Description

### Field of the invention

The present invention relates to medicated stick formulations containing 9-(2-hydroxyethoxymethyt)guanine, hereinafter called acyclovir, for the treatment of viral infections mostly caused by herpes simplex, localized in particular in the lip area.

### Prior art

The antiviral activity of acyclovir, its salts, esters or other derivatives as shown for instance in the English patent GB 1,523,865 (herein incorporated by reference) has long been known. A complete description of the antiviral activities and clinical properties of acyclovir is shown in J. J. O'Brien et al., Drugs 37, 233-309 (1989).

GB 1,523,865 also describes several examples of pharmaceutical compositions useful for administering acyclovir via different routes of administration: oral, parenteral, ocular or topical route.

Patent EP 44,543 refers in particular to the cutaneous route of administration and describes several acydovir formulations suitable for skin application as aqueous creams or in particular oil/water emulsions.

However, these and other formulations such as, for instance, those described in the international patent applications WO 95/16434, WO 94/05258 and WO 96/24 355 are inconvenient to be applied since dosage for treating herpes and in particular herpes labialis with acyclovir includes several and frequent applications of the topical formulation, especially in the early stage of development of herpes. This causes patients to have the formulation constantly at hand throughout the day, at least during the first few weeks of treatment

A solution to this problem, which is the object of the present invention, is the use of acyclovir formulations which can be suitably administered by topical applicators, also called medicated sticks. These formulations are not only extremely practical to use for the patient, but are also very effective and, compared to conventional creams or fatty ointments, can provide a cosmetic support which helps the patient also from a psychological point of view. The possibility of having a formulation convenient to use, easily at hand in any situation, leads to a more frequent and correct application of the active ingredient to the area involved and, as a result, to a more effective treatment of the disease.

In the description of the present invention, sticks are defined according to the commonly accepted English term as any preparations used in beauty treatments for lip make-up also known as sticks or more commonly known in beauty treatments by the name of lipsticks. When these preparations contain active ingredients, they are also known as medicated lipsticks. When these preparations are in the liquid form, they are then called roll-sticks or liquid lipsticks. Also in this case, when the formulations contain active ingredients, they are medicated liquid lipsticks.

The use of sticks for the treatment of herpetic disorders has already been described in patent EP 45,282. However, this patent only teaches the use of lipsticks made up of a mixture of polyethylene glycols for the administration of a combination of heparin or other sulphatized polymers with zinc salts. However, these formulations are not suitable for administering acyclovir.

### Summary

Therefore, an object of the present invention is a pharmaceutical composition suitable for the topical administration of acyclovir or a pharmaceutically acceptable derivative thereof (e.g. a salt or an ester thereof) by the use of solid sticks or roll-sticks.

The present invention relates to medicated sticks as set forth in the appended claims.

### Detailed description of the invention

Solid sticks are based on solid waxy excipients in which the active ingredient is dispersed. The mixture of excipients is such that the preparation can be uniformely distributed by lightly rubbing the stick on the damaged part. On the other hand, in the case of roll-on sticks, the composition includes an aqueous vehicle or another liquid vehicle in which the active ingredient is dissolved or dispersed. Thus, in this case the formulation is applied by means of a sphere which has the task of carrying the solution/suspension from the reservoir container to the surface to be treated.

Solid sticks have usually a soft consistency and are normally cylindrical with a base diameter of 8 to 10 mm and a length of about 3 to 4 cm. At the free end they can be rounded off in different ways, while the other flat end is adapted to the support of a case. The present solid stick comprises a pharmaceutically acceptable vehicle comprising the following hydrophobic excipients: at least one fatty substance other than waxes, at least one natural wax, optionally at least one paraffin substance (or synthetic wax), and may optionally further comprise at least one additive, such as fillers, preservatives and/or flavours.

The lipophilic vehicle of the present solid sticks is essentially free from water, and preferably also from C₂-C₆ polyhydric alcohols.

Greasiness, consistency, tenacity, flowability and most other characteristics of the finished products can be varied at will by per-cent and qualitative changes in the fatty ingredients.

In the present text, fatty substances are lipids, e.g. fats and fat-like substances. Fatty substances other than waxes are for instance selected among glycerides, fatty alcohols; fatty esters (i.e. esters of lower alcohols with fatty acids); lanolin and derivatives thereof such as lanolin alcohols (i.e. derived therefrom); sterols, and phospholipids.

In the present text, glycerides can be mono-, di-, tri- glycerides or mixtures thereof, either of natural (e.g. extractive) origin, or synthetic or semisynthetic glycerides.

In the present text, fatty acids are either saturated or unsaturated ones, in particular even-numbered, for instance of 10 to 36 carbon atoms, more particularly of 12 to 18 carbon atoms, such as stearic, oleic, cetyl, lauric, and palmitic acid.

Glycerides and glycerides based materials suitable as hydrophobic excipients for the present solid lipsticks are for instance oils, in particular castor oil (which may be optionally partially or totally replaced by hydrogenated castor oil) coconut oil, jojoba oil, sesame oil, corn oil, cottonseed oil, palm oil, kernel oil, rapeseed oil and olive oil, triglycerides such as stearin, cocoa butter, cocoa butter-like (i.e esters of glycerol with mixtures of oleic, palmitic and stearic acid, prepared by esterification of glycerol with mixtures of the corresponding fatty acids, or by inter-esterification of the corresponding glycerides) and medium chain triglycerides (Miglyol ^{R}).

Fatty alcohols can be either saturated or unsaturated ones, in particular even-numbered, for instance having from 10 to 36 carbon atoms, more particularly from 12 to 18 carbon atoms, such as cetyl, oleyl and stearyl alcohol.

Sterol is for instance cholesterol, cholestanol or other saturated sterols.

Phospholipids are phosphatidyl derivatives compatible for topical use, for instance esters of glycerophosporic acid with choline, inositol, or serine and with one or two fatty acids, e.g. alpha-lecithins.

Fatty esters are esters of fatty acids with low alcohols having in particular from 1 to 6 carbon atoms, for instance isopropyl miristate.

In the present text, unless otherwise stated waxes are the natural ones, i.e. those of natural origin.

Waxes are used to increase rigidity and consistency and include those of natural origin, such as white beeswax (a refining product obtained by treating natural yellow beeswax with oxidizing agents), spermaceti, candelilla, camauba waxes, bayberry wax and sugarcane wax.

Paraffin substances, or synthetic waxes can adjust some stick characteristics such as hardness, plasticity, flowability, greasiness and the like. Paraffins are hydrocarbons based substances, liquid, solid or semisolid, such as paraffins. petrolatum, paraffin oils (mineral oil) and the like. Examples of synthetic waxes are those derived from hydrogenated castor oil and coconut oil or from the other mentioned oils (i.e. esters of fatty acids derived from hydrogenated castor oil or coconut oil with sterols - such as cholesterol- or with fatty alcohols, generally even-numbered monohydric ones).

Other substances which may be added to solid sticks are fillers, which have the task of helping spreading of the composition to the lips. Insoluble inorganic substances including talc, calcium carbonate, zinc oxide and titanium dioxide, micronised magnesium or aluminum silicates may be used as fillers. Other fillers include water repellents such as silicon oils, which promote good adhesion to the lips. Preservatives, and more particularly antioxidants such as butylhydroxyanisol (BHA) and butylhydroxytoluene (BHT) propyl, octyl and dodecyl esters of gallic acid, tocopherol, ascorbyl palmitate, butyl, propyl and ethyl paraben or UV radiation filters such as Parsol^{R} (4-t-butyl-4'-methoxy-dibenzoylmethane) or derivatives of p-methoxycinnamic acid such as the esters thereof with C₂-C₆ alcohols known as Parsol-ultra^{R}, Parsol-MCX^{R}, can be used too.

The present solid or liquid sticks may optionally further comprise flavours or other additives conventional in the field of medicated lipsticks, in the usual amounts, and/or colouring agents or cover-up agents that provide cosmetic support.

In the present text, amounts of ingredients are expressed as by weight percentages referred to the total weight of the composition ingredients, which include both the pharmaceutically active ingredients (acyclovir, or esters or salts thereof, and optionally other active ingredients as specified hereinafter in the present text) and the ingredients of the vehicle, e.g. excipients, solvents, additives etc.

The present solid sticks may for instance contain the following amount of excipients, referred to the total weight of ingredients: from 20% to 90% by weight, more typically from 30% to 80% by weight of at least one fatty substance other than waxes; from 1% to 40% by weight, more preferably from 5% to 30% by weight of at least one wax; 5% to 35% by weight, more typically 10% to 30% by weight of at least one paraffin substance, if any. The amount of additives, if any, depends on the type thereof, and is for instance of from 0,1% to 10% by weight.

According to a particular embodiment of the present invention, the present solid stick comprises about 60% to 70% by weight of at least one fatty substance other than waxes, for instance castor oil (or hydrogenated castor oil) and about 10% to 30% by weight of at least one wax.

Preferred solid sticks according to the present invention comprise about 5% to 20% by weight of a wax and about 30% to 80% by weight of at least one fatty substance other than waxes, e.g. of triglycerides such as Miglyol ® (medium chain triclycerides, having in particular from 8 to 10 carbon atoms), for instance 50% - 80% by weight of such triglycerides combined with 10% - 20% of hydrogenated castor oil.

According to another particular embodiment of the present invention, the present solid stick invention comprises about 30% to 80% by weight of at least one fatty substance other than waxes, for instance castor oil (or hydrogenated castor oil) about 5% to 30% by weight of at least one wax, and about 10% to 30% by weight of a paraffin substance.

According to more particular embodiments of the present invention, the solid stick formulation comprises from about 30% to 60% by weight of an oil, preferably castor oil (or hydrogenated castor oil) and optionally from 5% to 45% by weight of another fatty substance other than waxes, selected for instance among lanolin, fatty alcohols, semisynthetic glycerides, such as for instance monoglycerides, and fatty esters (by weight percentages being also in this case referred to the total weight of ingredients).

The present solid stick composition may optionally comprise fillers, for instance in amounts of about from 5% to 10% by weight with respect to the total weight of ingredients, and/or antioxidants, e.g. in amounts of about 0.1% to 10% by weight referred to the total ingredients weight.

As far as excipients suitable for roll-sticks are concemed, these differ from the above ones because of the liquid consistency, which can be more or less viscous depending on the thickening agents they contain or on the solvent characteristics. The liquid vehicle is alternatively an aqueous vehicle preferably containing thickening agents, or alternatively is an alcohol base, comprising at least one pharmaceutically acceptable alcohol, all these vehicles optionally further containing one or more additives.

The thickening agent used in the present aqueous vehicle is for instance a hydrophilic one, either water soluble or water swellable, such as gums, alginates, acrylic polymers such as carboxypolymethylene (Carbomer ^{R}, i.e. high molecular weight polymers or acrylic acid cross linked with either allylsucrose ot allylethers of pentaerythrol), acrylic copolymers, cellulose derivatives such as cellulose ethers (e.g. hydroxyethyl or hydroxy propyl cellulose, sodium carboxymethyl cellulose), and polyvinyl alcohols, optionally combined with one or more additives, such as plasticizers, for instance triethylcitrate or dioctylacetate, non-ionic surface active agents, or water miscible polyhydric alcohols, having e.g. 2 to 6 carbon atoms.

The present aqueous vehicle may for instance contain at least one thickening agent, for instance a glyceryl ester of fatty acids, or a hydrophilic thickening agent, combined with a surfactant agent and/or with a water miscible organic co-solvent, for instance selected among polyhydric alcohols, e.g. with 2 to 6 carbon atoms (such as ethylene glycol and propylene glycol), diethylene glycol monoethyl ether, and mixtures thereof.

The present aqueous compositions are typically free from lipohilic components selected among fat substances such as waxes and lanolin, and hydrocarbon based hydrophobic substances such as paraffin. Preferably, they are also free from dimethylsulphoxide and /or from glycerol formal.

Preferred aqueous formulations of the present invention contain water (e.g. about 20% to 30% by weight); at least one thickening agent, for instance a hydrophilic one such as Carbomer ^{R}, e.g. Carbomer 940, (e.g. in amounts of about 0.05% to 1% by weight, preferably 0.1% - 0.5% by weight) and preferably also at least one organic co-solvent, such as diethylene glycol monoethylether (Transcutol^{R})(e.g. in amounts of about 10% to 50 % by weight, preferably of about 20% to 40% by weight).

Preferably, diethylene glycol monoethylether is combined with a C₂-C₆ polyhydric alcohol, such as propylene glycol, (e.g. in amounts of about 10% to 50% by weight, preferably of 20% to 40% by weight),

Some aqueous compositions containing thickening agents according to the present invention contain at least 50% by weight of water and less than 30% by weight of said co-solvents.

According to a particular embodiment of the present invention, the aqueous formulation comprises water (e.g. about 70% to 95% by weight); a thickening agent (e.g. 1% to 10%, preferably 3-5% by weight), such as glyceryl monostearate; and preferably at least one water miscible co-solvent, in amounts usually lower than 30% by weight with respect to the total weight of the composition, usually up to 10% by weight, (e.g. from 1% to 10% by weight), for instance a C₂-C₆ polyhydric alcohol such as ethylene glycol, propylene glycol or mixtures thereof, e.g. in amount of about 3-6% by weight.

The pharmaceutically acceptable alcohols which can be used in roll-stick vehicles are more particularly C₂-C₁₀ alcohols, for instance ethyl alcohol, isopropyl alcohol or benzyl alcohol, and the like, preferably on aqueous solution in amounts for instance of about from 10% to 40% by weight with respect to the total ingredients weight and optionally about 0.1% to 5% by weight of at least one additive, such as flavours and preservatives.

Some liquid compositions according to the present invention (more particularly aqueous compositions) may contain high boiling solvents, such as polyethylene glycols (PEG) with a medium molecular weight lower that 1000 (e.g. between 200 and 600), such as for instance PEG 200, 600, and mixtures thereof, or derivatives thereof, such as mono- or di-esters with fatty acids, such as lauric acid and ricinoleic acid, and optionally other additives.

The solutions to be filled into roll-sticks are prepared simply by dissolving the formulation ingredients in a selected solvent to which a surface-active agent is usually added and acyclovir or a derivative thereof (e.g. a salt or a ester thereof), optionally combined with one or more other active ingredients, is then incorporated into the resulting vehicle at a weight/weight concentration varying from 0.5 to 10%, preferably from 2 to 5%. The resulting mixture is then poured into roll-stick reservoirs, with a capacity e.g. of 2 to 10 ml of composition per each stick.

Acyclovir may be dispersed, partially dissolved or dissolved in the present compositions. In the case of dispersions, micronised form (mean particle size less than 10 µm -micrometers-, for instance between 1 and 5 µm), while the product to be dissolved may have a mean particle size larger than 10 µm, for instance from 10 µm to 150 µm.

The amount of acyclovir or pharmaceutically acceptable derivatives thereof (e.g. salts or esters thereof) included in the compositions for lip applicators according to the present invention, either the solid or the liquid ones, may vary from 0.5 to 10%, preferably from 2% to 5% by weight with respect to the total weight of the ingredients used.

The present lipsticks compositions, either the solid sticks or the roll-sticks, are topically applied for instance from 1 to 10 times a day, e.g. at least five times a day, while symptoms persist, for periods generally of from 1 to 10 days. Daily dosage of acyclovir topically administered to humans by means of the present lipstick compositions, either the solid sticks or the roll-sticks ranges for instance from 5 to 100 mg of acyclovir.

The methods of preparation are the classic methods commonly used in the cosmetic industry, such as for instance those described by P. G. I. Lauffer in M. S. Balsam and E. Sagarin, COSMETICS, Science and Technology, Vol. 1, 2nd Ed., pages 365-392, Wiley Interscience, 1972.

The present compositions for solid lipsticks are for instance prepared by incorporating under stirring the active ingredient (i.e. acyclovir, or a salt or ester thereof, and optionally a further active ingredient) into a melted mixture of fatty excipients (i.e. fatty substances other than waxes, waxes and optionally paraffin substances), melted for instance at about 50°C to 90°C, usually at about from 60°C to 80°C, optionally adding either before or after the addition of the active ingredient, with one or more additives, filling lipsticks moulds (having e.g. a capacity of about 2-4 grams) with the resulting mixture, then cooling to solidification, usually to room temperature (e.g. 20°/25°C).

It has also been found - and this is a further aspect of the present invention - that there is improved curative activity in labial herpes infection when vitamin A is combined with topically administered acyclovir. The presence of vitamin A as retinol or derivatives thereof, e.g. esters thereof with C₁-C₆ carboxylic acids or with fatty acids, in particular retinol acetate or retinol palmitate, or retinoic acid, in the described pharmaceutical compositions helps lesion cicatrization by significantly reducing lesion healing time.

Vitamin A as retinol or esters thereof is used in concentrations varying from 0.5 to 10%, preferably from 1 to 3% by weight, with respect to the total weight of the composition. Unit doses are often expressed as international units (I.U.), one I.U. being equal to 0.3 µg of retinol. Preferred daily doses are within a range of 10,000 to 50,000 I.U./die.

Acyclovir or derivatives thereof included in the pharmaceutical compositions which are an object of the present invention may also be combined with other antiviral agents, e.g. guanine derivatives such as valacyclovir, gancyclovir, famciclovir, pencyclovir, 9-(1,3-dihydroxy-2-propoxymethyl)guanine, acyclovir derivatives such as phosphate derivatives, phosphotriester derivatives, valine ester derivatives and the like; vidarabine, cytarabine, phosphonoacetic acid, phosphonoformic acid, idoxuridine, trifluridine, edoxudine, brovavir, flacitabine, rociclovir and the like. In some cases these combinations help in increasing the antiherpetic activity of acyclovir.

Even though the formulations described can be used for any topical treatment of herpetic disease, such as for instance a disease of nasal mucous membranes, they are, nevertheless, particularly indicated for the treatment and prevention of diseases caused by herpes labialis.

There are hereinbelow reported some examples which are only meant to better describe the object of the invention and show its advantages and applicability, without limiting the same.

### Compositions for solid medicated lipsticks

### EXAMPLE 1

100 g of composition contain:

| | |
|---|---|
| Acydovir | 3.00 g |
| Retinol palmitate | 3.00 g |
| Castor oil | 60.00 g |
| Camauba wax | 10.00 g |
| Semisynthetic glycerides | 9.00 g |
| Parsol^{R} | 7.00 g |
| Beeswax | 7.00 g |
| Butylhydroxyanisol (BHA) | 0.10 g |
| Glycyrrhizin ammonium | 0.10 g |
| Vanilla flavour | 0.80 g |

The composition was prepared dissolving camauba wax, semisynthetic glycerides and beeswax under stirring in castor oil heated to 80°C. After dissolution, the flavours, acyclovir, Parsol^{R}, retinol palmitate and BHA were added maintaining the mixture at 80°C. Special plastic containers fitted into moulds were filled with the mixture still kept at 80°C to a dose of 2.5 g. The sticks were cooled to ambient temperature, then removed and finally closed with caps.

### EXAMPLE 2

5% micronised acyclovir was dispersed under stirring at 60°C up to homogeneous dispersion in a fatty phase made up of:

| | |
|---|---|
| Camauba wax | 10% |
| Beeswax | 15% |
| Lanolin | 5% |
| Cetyl alcohol | 5% |
| Hydrogenated castor oil | 60% |

The melted mass containing acyclovir was filled into 3-g cylindrical containers for sticks.

### EXAMPLE 3

Fats made up of a waxy mixture having the following composition:

| | |
|---|---|
| Beeswax | 30% |
| Mineral oil | 20% |
| Isopropyl myristate | 5% |
| Polyethylene glycol 400 | 5% |
| Castor oil | 35% |

were melted.

5% micronised acyclovir was added to the melted mass under stirring and, after homogenisation, the mass was filled into appropriate containers for 3.5 g lipsticks.

### EXAMPLE 4

A preparation according to the preceding example, containing 5% acyclovir and:

| | |
|---|---|
| Castor oil | 30% |
| Mineral oil | 15% |
| Beeswax | 15% |
| Paraffin | 10% |
| Camauba wax | 15% |
| Silicon oil | 10% |

### EXAMPLE 5

Preparation in accordance with the method in Example 2. The active ingredient acyclovir (5%) was added to a vehicle having the following composition:

| | |
|---|---|
| Glyceryl monostearate (Tegin 515^{R}) | 42% |
| Castor oil | 36% |
| Mineral oil | 6% |
| Paraffin | 6% |
| Camauba wax | 5% |

### EXAMPLE 6

A preparation was obtained according to the method of example 2. The active ingredient acyclovir (5%) was added to a vehicle having the following composition:

| | |
|---|---|
| Hydrogenated castor oil | 12% |
| Miglyol ^{R} | 65% |
| Beeswax | 18% |

### EXAMPLE 7

A preparation was obtained according to the method of Example 2. The active ingredients acyclovir (5%) and vitamin A (3%) were added to a vehicle having the following composition:

| | |
|---|---|
| Hydrogenated castor oil | 12% |
| Miglyol ^{R} | 62% |
| Beeswax | 17.8% |
| BHT | 0.2% |

### Composition for medicated roll-sticks

### EXAMPLE 8

A solution containing 3% sodium acyclovir in a vehicle made up of:

| | |
|---|---|
| Gtyceryl monostearate | 4.0% |
| Ethylene glycol | 4.0% |
| Propylene glycol | 1.5% |
| Lavender essence | 0.5% |
| Water | 87.0% |

was prepared.

The solution was filled into 10-ml roll-sticks.

### EXAMPLE 9

Acyclovir (5%) was added to a vehicle having the following by weight percent composition:

| | |
|---|---|
| Carbomer ^{R} 940 | 0.2% |
| Transcutol ^{R} (diethylene glycol monoethylether) | 30% |
| Propylene glycol | 40% |
| Sodium hydroxide | 0.05% |
| Water up to | 100 |

The solution was filled into 10 ml roll-sticks.

### EXAMPLE 10

Acyclovir (5%) and vitamin A (3%) were added to a vehicle having the following by weight percent composition:

| | |
|---|---|
| Carbomer ^{R} 940 | 0.2% |
| Transcutol ^{R} | 30% |
| (Diethylene glycol monoethylether) | |
| Propylene glycol | 40% |
| Sodium hydroxide | 0.05% |
| Water up to | 100 |

The solution was filled into 10 ml roll-sticks.

### Evaluation of activity and conveniency of use

### EXAMPLE 11

Three formulations made up of:
A) a commercial topical 5% acyclovir formulation as a cream (Zovirax^{R}),
B) a stick formulation containing 5% acydovir prepared in accordance with the method described in Example 2,
C) a stick formulation containing 3% acydovir and 3% vitamin A, prepared according to Example 1,
were tested in 30 patients with herpes labialis in a clinical study.

The patients, usually suffering from lip herpes, were divided into 3 groups of 10 persons and each group received one of the three formulations (A, B, C). The patients were asked to apply the formulation assigned to them as they were used to, and were given at the same time a questionnaire in which they had to report for a period of 10 days from the beginning of the treatment
1) the number of daily applications;
2) a personal evaluation, using a scale from 1 to 3, of the comfort of use;
3) a second subjective evaluation, using a scale from 1 to 5, of the progress of healing.

The results of the study are summarized in the two tables enclosed.

**TABLE 1**

| Evaluation of the application frequency and comfort of use of formulations containing acyclovir | | |
|---|---|---|
| Groups | Mean daily applications | Comfort of use (scale 1 to 3) (*) |
| A (cream - Zovirax^{R}) | 4.3 | 1.5 |
| | | |
| B (stick) | 6.8 | 2.8 |
| | | |
| C (stick with vitamin A) | 7.1 | 2.9 |

| | | |
|---|---|---|
| Note (*): 1 = lowest, 3 = highest | | |

**TABLE 2**

| Effectiveness of the tested compositions Evaluation of the activity of compositions containing acyclovir | | | | | | | |
|---|---|---|---|---|---|---|---|
| Group | Day | | | | | | |
| | 1 | 2 | 3 | 4 | 5 | 7 | 10 |
| A Scale (*) | 1.0 | 1.0 | 1.5 | 2.1 | 3.2 | 3.5 | 4.5 |
| B " | 1.0 | 1.5 | 2.8 | 3.5 | 4.0 | 4.2 | 4.6 |
| C " | 1.0 | 1.8 | 3.1 | 4.1 | 4.5 | 4.8 | 5.0 |

| | | | | | | | |
|---|---|---|---|---|---|---|---|
| Note (*): Scale 1 to 5: 1 = no activity. 5 = complete healing | | | | | | | |

The results summarized in the above reported two tables clearly show the following indications:
1) The sticks are more convenient to use than the usual cream formulations (Table 1, groups B and C compared to A).
2) The stick was found to be more effective than the cream as it reduces healing time (Table 2, groups B and C compared to A).
3) Better results are obtained when acydovir is combined with vitamin A as retinol palmitate in a stick (Table 2, group C compared to groups A and B).

### EXAMPLE 12

The following study is performed to evaluate the therapeutic efficacy and convenience of use for lipstick and roll-on formulations prepared according to the present invention. The formulation are as follows:
A) a commercial topical 5% acyclovir formulation as a cream (Zovirax ^{R});
B) a stick formulation containing 5% acyclovir prepared according to Example 2;
C) a stick containing formulation as in B), containing in addition 3% Vitamin A;
D) a stick formulation containing 3% acyctovir prepared according to Example 1;
E) a stick formulation as in D), containing in addition 3% Vitamin A;
F) a stick formulation as in E), containing in addition colour and/or cover-up additives;
G) a roll-on formulation containing 3% acyclovir prepared according to Example 8;
H) a roll-on formulation containing 3% acyclovir and 3% vitamin A, prepared according to Example 8; and
I) a roll-on formulation as in H), containing in addition colour and/or cover-up additives.

The formulations are tested in a randomized patient population suffering from Herpes labialis infection in a clinical study.

The patients are divided into groups, each group receiving one of the above-described formulations or a control formulation not containing acyclovir or Vitamin A. Groups F and I (and control groups) comprise female patients.

Within each group, half of the patients are asked to apply the formulation assigned to them as they are accostumed, and are given at the same time a questionnaire in which they are asked to report the following items for a period of 10 days from the beginning of the treatment:
1) the number of daily applications;
2) a personal evaluation, using a scale from 1 to 3, of the convenience of use; and
3) a second subjective evaluation, using a scale from 1 to 5, of the progress of healing.

The second half of each group of patients are asked to apply the formulation five times a day. They are given at the same time a questionnaire in which they are asked to report the following items for a period of 10 days from the beginning of the treatment:
1) the time of each application;
2) a personal evaluation, using a scale from 1 to 3, of the convenience of use; and
3) a second subjective evaluation, using a scale from 1 to 5, of the progress of healing.

The study may also involve evaluation of the progress of healing by a physician.

The results of this study are evaluated using a statistical method such as chi-square analysis or t-test to determine if the differences between the groups are statistically significant.

The results demonstrate that the formulations of the invention provide enhanced therapeutic efficacy and improved convenience of use with respect to topical acyclovir cream treatment.

## Claims

1. A medicated stick for topical administration containing:
i) acyclovir or a pharmaceutically acceptable derivative thereof as the sole active ingredient, or
ii) said acyclovir being optionally associated with an active ingredient selected from the group consisting of: retinol or a pharmaceutically acceptable derivative thereof, and/or at least one other antiviral active ingredient,
said stick being solid or liquid (roll-stick).

2. The solid medicated stick according to claim 1, **characterized in that** it contains a vehicle comprising at least one fatty substance other than waxes, at least one natural wax, optionally at least one paraffin substance (or synthetic wax), and optionally at least one additive.

3. The solid medicated stick according to claim 2, **characterized in that** the fatty substance is selected among glycerides, fatty alcohols, esters of lower alcohols with fatty acids, lanolin or alcohols derived therefrom, sterols and phospholipids.

4. The solid medicated stick according to claim 3, **characterized in that** the glycerides are selected among castor oil (optionally partially or totally replaced by hydrogentated castor oil), jojoba oil, sesame oil, corn oil, cottonseed oil, palm oil, kernel oil, rapeseed oil, olive oil, coconut oil, stearin, cocoa butter, cocoa butter-like; fatty alcohols are selected among cetyl, oleyl and stearyl alcohol; sterol is cholesterol; phospholipids are alpha-lecitihins; fatty esters are esters of fatty acids with low alcohols of from 1 to 6 carbon atoms.

5. The solid medicated stick according to claim 1, wherein the wax is selected among white beeswax, camauba wax, spermaceti, candelilla wax, bayberry wax and sugarcane wax.

6. The solid medicated stick according to claim 1, **characterized in that** the paraffin substance is selected among paraffins, petrolatum, and paraffin oils.

7. The solid medicated stick according to claim 2, **characterized in that** the additive is selected among fillers, preservatives and flavours.

8. The solid medicated stick according to claim 7, **characterized in that** the filler is selected among talc, calcium carbonate, zinc oxide, titanium dioxide, magnesium silicates, micronised aluminum, and silicon oils; and the preservative is selected among butylhydroxyanisol, butylhydroxytoluene, 4-t-butyl-4'-methoxy-dibenzoylmethane, esters of p-methoxycinnamic acid with C₁-C₆ alcohols; propyl, octyl or dodecyl esters of gallic acid, tocopherol, ascorbyl, palmitate, and butyl, propyl or ethyl paraben.

9. The solid medicated stick according to claim 2, **characterized in that** the amount of fatty substance other than waxes is from 20% to 90% by weight, the amount of wax is from 1% to 40% by weight, the amount of paraffin substance is from 5% to 35% by weight, and the amount of additive, if any, is from 0.1% to 10% by weight, percent amounts being referred to the total weight of the ingredients of the composition.

10. The solid medicated stick according to claim 2, **characterized in that** it comprises from 60% to 70% by weight of at least one fatty substance other than wax and about from 10% to 30% by weight of a wax with respect to the total weight of ingredients of the composition.

11. The solid medicated stick according to claim 2, **characterized in that** it comprises from 30% to 80% by weight of at least one fatty substance other than waxes, from 55 to 30% by weight of at least one wax, and from 10% to 30% by weight of at least one paraffin substance.

12. The solid medicated stick according to claim 10 or 11, **characterized in that** the solid stick comprises from 30% to 60% by weight of castor oil, and optionally 5% to 45% by weight of another fatty substance other than wax, selected among lanolin, fatty alcohol, semisynthetic glycerides, and esters of fatty acids with C1-C6 alcohols, percentages by weight being referred to the total weight of ingredients of the composition.

13. The solid medicated stick according to claim 2, **characterized in that** it comprises 5% to 20% by weight of a wax and 30% to 80% by weight of triglycerides.

14. The solid medicated stick according to claim 13, **characterized in that** it comprises 5% to 20% by weight of a wax, 50% to 80% by weight of medium chain triglycerides and 10% to 20% of hydrogenated castor oil.

15. The liquid medicated stick according to claim 1, **characterized in that** it comprises 20% to 30% by weight of water, 0.05% to 1% by weight of at least one hydrophilic thickening agent, and at least 10% to 50% by weight of diethylene glycol monoethylether.

16. The liquid medicated stick according to claim 15, comprising 20% to 40% by weight of diethylene glycol monoethylether and 20% to 40% by weight of a C₂-C₆ polyhydric alcohol.

17. The liquid medicated stick according to claim 15 or 16, **characterized in that** the thickening agent is selected among gums, alginates, acrylic polymers or copolymers, cellulose derivatives, polyvinyl alcohols, and glyceryl esters, optionally at least one plasticizer selected among triethylcitrate, dioctylacetate, non-ionic surface active agents and water miscible polyhydric alcohols.

18. The liquid medicated stick according to any one of claims from 15 to 17, wherein the thickening agent is carboxypolymethylene.

19. The liquid medicated stick according to claim 1, containing an aqueous vehicle comprising water, at least one thickening agent, combined with a water miscible organic co-solvent, **characterized in that** it contains at least 50% by weight of water and less than 30% by weight of said co-solvent, and by being free from lipophilic substances selected among waxes, lanolin, and hydrocarbon based hydrophobic substances.

20. The liquid medicated stick according to claim 19, containing from 70% to 95% by weight of water, from 1% to 10% by weight of a hydrophobic thickening agent, and less than 30% by weight of a water miscible organic co-solvent, referred to the total weight of the composition.

21. The liquid medicated stick according to claim 19, wherein the hydrophobic thickening agent is glyceryl monostearate, and the water miscible organic co-solvent is a C₂-C₆ polyhydric alcohol.

22. The liquid medicated stick according to claim 1, **characterized in that** it is an aqueous solution containing at least one C₂-C₁₀, alcohol and optionally at least one additive.

23. The liquid medicated stick according to claim 22, comprising the alcohol selected among ethyl alcohol, isopropyl alcohol and benzyl alcohol.

24. The liquid medicated stick according to claim 22, wherein the alcohol is in amount of from 10% to 40% by weight with respect to the total ingredients weight, and 0.1% to 5% by weight of additive.

25. The liquid medicated stick according to claim 1, **characterized in that** it comprises an aqueous vehicle comprising polyethylene glycols with a medium molecular weight lower than 1000 or mono- o di-esters thereof with fatty acids.

26. The liquid medicated stick according to claim 25, wherein polyethylene glycols (PEG) are selected among PEG 200, PEG 600 and mixtures thereof.

27. The medicated stick according to any one of claims from 1 to 26, wherein the retinol derivative is an ester thereof selected between retinol acetate and retinol palmitate, or retinoic acid.

28. The medicated stick according to any one of claims from 1 to 27, comprising retinol or a derivative thereof in amounts of from 0.5% to 10% by weight with respect to the total ingredients weight of the medicated stick.

29. The medicated stick according to any one of claims from 1 to 28, comprising retinol or an ester thereof in amounts of from 1% to 3% by weight with respect to the total weight of the medicated stick ingredients.

30. The medicated stick according to any one of claims 1-29, wherein said antiviral active ingredient is selected among valacyclovir, gancyclovir, famciclovir, pencyclovir, 9-(1,3-dihydroxy-2-propylmethyl)guanine, acyclovir phosphate derivatives, phosphotriester derivatives or valine ester derivatives, vidabarin, cytarabin, phosphonoacetic acid, phosphonoformic acid, idoxuridine, trifluridine, edoxudine, brovavir, flacitabine and rociclovir.

31. The medicated stick according to any one of claims from 1 to 30, **characterized in that** the amount of acyclovir or derivative thereof therein contained is from 0.5% to 10% by weight with respect to the total weight of ingredients.

32. The medicated stick according to claim 31, **characterized in that** said amount is from 2% to 5% by weight.

33. Use of acyclovir or a pharmaceutically acceptable derivative thereof for the preparation of a pharmaceutical composition for the topical treatment of a viral disease, **characterized in that** said composition is a medicated stick as defined in anyone of claims from 1 to 32.

34. The use according to claim 33, wherein said disease is herpes labialis.

35. A process for preparing a solid medicated stick according to any one of claims from 1 to 14, which comprises incorporating under stirring acyclovir or an ester or a salt thereof and optionally the other therapeutically active ingredients into the melted mass of liphophilic excipients, optionally adding one or more additives, filling the resulting melted mass into moulds for lipsticks, and cooling to solidification.

36. A process for preparing a liquid medicated stick according to any one of claims from 15 to 32, which comprises dissolving the formulation ingredients in a selected solvent to which a surface-active agent is optionally added, then pouring the resulting mixture into roll-on stick reservoirs.

37. A topical delivery device comprising a composition as claimed in anyone of claims from 1 to 32, and a support for solid sticks or a reservoir for roll-on sticks.

## Patentansprüche

1. Arzneihaltiger Stift zur topischen Anwendung, der folgendes enthält:
(i) Acyclovir oder ein pharmazeutisch annehmbares Derivat davon als einzigen Wirkstoff oder
(ii) Acyclovir, das gegebenenfalls verbunden ist mit einem Wirkstoff, ausgewählt aus Retinol oder einem pharmazeutisch annehmbaren Derivat davon und/oder mindestens einem anderen antiviral wirksamen Bestandteil, wobei der Stift fest oder flüssig (Rollstift) ist.

2. Fester arzneihaltiger Stift gemäss Anspruch 1, **dadurch gekennzeichnet, dass** er einen Träger enthält, der mindestens eine von Wachsen unterschiedliche, fettige Substanz, mindestens ein natürliches Wachs, gegebenenfalls mindestens eine Paraffinsubstanz (oder ein synthetisches Wachs) und gegebenenfalls mindestens einen Zusatzstoff umfasst.

3. Fester arzneihaltiger Stift gemäss Anspruch 2, **dadurch gekennzeichnet, dass** die fettige Substanz ausgewählt ist aus Glyceriden, Fettalkoholen, Estern niederer Alkohole mit Fettsäuren, Lanolin oder davon abgeleiteten Alkoholen, Styrolen und Phospholipiden.

4. Fester arzneihaltiger Stift gemäss Anspruch 3, **dadurch gekennzeichnet, dass** die Glyceride ausgewählt sind aus Castoröl (gegebenenfalls teilweise oder vollständig durch hydriertes Castoröl ersetzt), Jojobaöl, Sesamöl, Maisöl, Baumwollsamenöl, Palmöl, Kernöl, Rapssaatöl, Olivenöl, Kokosnussöl, Stearin, Kakaobutter und kakaobutterähnlichen; die Fettalkohole sind ausgewählt aus Cetyl-, Oleyl- und Stearylalkohol; das Sterol ist Cholesterol, die Phospholipide sind α-Lecithine, die fettigen Ester sind Ester von Fettsäuren mit niederen Alkoholen mit 1-6 Kohlenstoffatomen.

5. Fester arzneihaltiger Stift gemäss Anspruch 1, worin das Wachs ausgewählt ist aus weissem Bienenwachs, Carnaubawachs, Spermaceti, Candelillawachs, Gagelstrauchwachs und Zuckerrohrwachs.

6. Fester arzneihaltiger Stift gemäss Anspruch 1, **dadurch gekennzeichnet, dass** die Paraffinsubstanz ausgewählt ist aus Paraffinen, Petrolatum und Paraffinölen.

7. Fester arzneihaltiger Stift gemäss Anspruch 2, **dadurch gekennzeichnet, dass** der Zusatzstoff ausgewählt ist aus Füllstoffen, Konservierungsmitteln und Duftstoffen.

8. Fester arzneihaltiger Stift gemäss Anspruch 7, **dadurch gekennzeichnet, dass** der Füllstoff ausgewählt ist aus Talk, Calciumcarbonat, Zinkoxid, Titandioxid, Magnesiumsilicaten, mikronisiertem Aluminium und Siliconölen; und das Konservierungsmittel ist ausgewählt aus Butylhydroxyanisol, Butylhydroxytoluol, 4-t-Butyl-4'-methoxydibenzoylmethan, Estern von p-Methoxyzimtsäure mit C₁₋₆-Alkoholen; Propyl-, Octyl- oder Dodecylestern von Gallsäure, Tocopherol, Ascorbyl, Palmitat und Butyl-, Propyl- oder Ethylparaben.

9. Fester arzneihaltiger Stift gemäss Anspruch 2, **dadurch gekennzeichnet, dass** die Menge an von Wachsen unterschiedlicher fettiger Substanz 20-90 Gew.%, die Menge an Wachs 1-40 Gew.%, die Menge an Paraffinsubstanz 5-35 Gew.% und die Menge an Zusatzstoff, sofern vorhanden, 0,1-10 Gew.% beträgt, wobei die prozentualen Mengen auf das Gesamtgewicht der Bestandteile der Zusammensetzung bezogen sind.

10. Fester arzneihaltiger Stift gemäss Anspruch 2, **dadurch gekennzeichnet, dass** er 60-70 Gew.% mindestens einer von Wachs unterschiedlichen fettigen Substanz und etwa 10 bis 30 Gew.% eines Wachses, bezogen auf das Gesamtgewicht der Bestandteile der Zusammensetzung, umfasst.

11. Fester arzneihaltiger Stift gemäss Anspruch 2, **dadurch gekennzeichnet, dass** er 30-80 Gew.% mindestens einer von Wachsen unterschiedlichen fettigen Substanz, 55-30 Gew.% mindestens eines Wachses und 10-30 Gew.% mindestens einer Paraffinsubstanz umfasst.

12. Fester arzneihaltiger Stift gemäss Anspruch 10 oder 11, **dadurch gekennzeichnet, dass** der feste Stift 30-60 Gew.% Castoröl und gegebenenfalls 5-45 Gew.% einer anderen, von Wachs unterschiedlichen, fettigen Substanz, ausgewählt aus Lanolin, Fettalkohol, halbsynthetischen Glyceriden und Estern von Fettsäuren mit C₁₋₆-Alkoholen umfasst, die Gewichte beziehen sich auf das Gesamtgewicht der Bestandteile der Zusammensetzung.

13. Fester arzneihaltiger Stift gemäss Anspruch 2, **dadurch gekennzeichnet, dass** er 5-20 Gew.% eines Wachses und 30-80 Gew.% Triglyceride umfasst.

14. Fester arzneihaltiger Stift gemäss Anspruch 13, **dadurch gekennzeichnet, dass** er 5-20 Gew.% des Wachses, 50-80 Gew.% mittelkettiger Triglyceride und 10-20 % hydriertes Castoröl umfasst.

15. Flüssiger arzneihaltiger Stift gemäss Anspruch 1, **dadurch gekennzeichnet, dass** er 20-30 Gew.% Wasser, 0,05-1 Gew.% mindestens eines hydrophilen Verdickungsmittels und mindestens 10-50 Gew.% Diethylenglykolmonoethylether umfasst.

16. Flüssiger arzneihaltiger Stift gemäss Anspruch 15, der 20-40 Gew.% Diethylenglykolmonoethylether und 20-40 Gew.% eines mehrwertigen C₂₋₆-Alkohols umfasst.

17. Flüssiger arzneihaltiger Stift gemäss Anspruch 15 oder 16, **dadurch gekennzeichnet, dass** das Verdickungsmittel ausgewählt ist aus Gummis, Alginaten, Acrylpolymeren oder -Copolymeren, Cellulosederivaten, Polyvinylalkoholen und Glycerylestern, gegebenenfalls mindestens einem Weichmacher, ausgewählt aus Triethylcitrat, Dioctylacetat, nicht-ionischen Tensiden und wassermischbaren mehrwertigen Alkoholen.

18. Flüssiger arzneihaltiger Stift gemäss mindestens einem der Ansprüche 15 bis 17, worin das Verdickungsmittel Carboxypolymethylen ist.

19. Flüssiger arzneihaltiger Stift gemäss Anspruch 1, der einen wässrigen Träger enthält, der Wasser und mindestens ein Verdickungsmittel in Kombination mit einem wassermischbaren organischen Co-Lösungsmittel umfasst, **dadurch gekennzeichnet, dass** er mindestens 50 Gew.% Wasser und weniger als 30 Gew.% des Co-Lösungsmittels enthält und frei von lipophilen Substanzen, ausgewählt aus Wachsen, Lanolin und hydrophoben Substanzen auf Kohlenwasserstoffbasis, ist.

20. Flüssiger arzneihaltiger Stift gemäss Anspruch 19, der 70-95 Gew.% Wasser, 1-10 Gew.% eines hydrophoben Verdickungsmittels und weniger als 30 Gew.% eines wassermischbaren organischen Co-Lösungsmittels, bezogen auf das Gesamtgewicht der Zusammensetzung, enthält.

21. Flüssiger arzneihaltiger Stift gemäss Anspruch 19, worin das hydrophobe Verdickungsmittel Glycerylmonostearat ist und das wassermischbare organische Co-Lösungsmittel ist ein mehrwertiger C₂₋₆-Alkohol.

22. Flüssiger arzneihaltiger Stift gemäss Anspruch 1, **dadurch gekennzeichnet, dass** er eine wässrige Lösung, die mindestens einen C₂₋₁₀-Alkohol und gegebenenfalls mindestens einen Zusatzstoff enthält, darstellt.

23. Flüssiger arzneihaltiger Stift gemäss Anspruch 22, der einen Alkohol, ausgewählt aus Ethylalkohol, Isopropylalkohol und Benzylalkohol, umfasst.

24. Flüssiger arzneihaltiger Stift gemäss Anspruch 22, worin der Alkohol in einer Menge von 10-40 Gew.% in bezug auf das Gewicht aller Bestandteile, und der Zusatzstoff in einer Menge von 0,1-5 Gew.% vorhanden ist.

25. Flüssiger arzneihaltiger Stift gemäss Anspruch 1, **dadurch gekennzeichnet, dass** er einen wässrigen Träger umfasst, der Polyethylenglykole mit einem mittleren Molekulargewicht von weniger als 1.000 oder Mono- oder Diester davon mit Fettsäuren umfasst.

26. Flüssiger arzneihaltiger Stift gemäss Anspruch 25, worin die Polyethylenglykole (PEG) ausgewählt sind aus PEG 200, PEG 600 und Mischungen daraus.

27. Arzneihaltiger Stift gemäss mindestens einem der Ansprüche 1 bis 26, worin das Retinolderivat ein Ester davon ist, ausgewählt aus Retinolacetat und Retinolpalmitat, oder Retinolsäure.

28. Arzneihaltiger Stift gemäss mindestens einem der Ansprüche 1 bis 27, der Retinol oder ein Derivat davon in einer Menge von 0,5-10 Gew.%, bezogen auf das Gesamtgewicht der Bestandteile des arzneihaltigen Stifts, umfasst.

29. Arzneihaltiger Stift gemäss mindestens einem der Ansprüche 1 bis 28, der Retinol oder einen Ester davon in einer Menge von 1-3 Gew.%, bezogen auf das Gesamtgewicht der Bestandteile des arzneihaltigen Stifts, umfasst.

30. Arzneihaltiger Stift gemäss mindestens einem der Ansprüche 1 bis 29, worin der antiviral aktive Bestandteil ausgewählt ist aus Valacyclovir, Gancyclovir, Famciclovir, Pencyclovir, 9-(1,3-Dihydroxy-2-propylmethyl)guanin, Acyclovirphosphatderivaten, Phosphotriesterderivaten oder Valinesterderivaten, Vidabarin, Cytarabin, Phosphonoessigsäure, Phosphonoameisensäure, Idoxuridin, Trifluridin, Edoxudin, Brovavir, Flacitabin und Rocyclovir.

31. Arzneihaltiger Stift gemäss mindestens einem der Ansprüche 1 bis 30, **dadurch gekennzeichnet, dass** das Acyclovir oder ein Derivat davon in einer Menge von 0,5-10 Gew.%, bezogen auf das Gesamtgewicht der Bestandteile, enthalten ist.

32. Arzneihaltiger Stift gemäss Anspruch 31, **dadurch gekennzeichnet, dass** die Menge 2-5 Gew.% beträgt.

33. Verwendung von Acyclovir oder eines pharmazeutisch annehmbaren Derivats davon zur Herstellung einer pharmazeutischen Zusammensetzung zur topischen Behandlung einer viralen Erkrankung, **dadurch gekennzeichnet, dass** die Zusammensetzung ein arzneihaltiger Stift gemäss mindestens einem der Ansprüche 1 bis 32 ist.

34. Verwendung gemäss Anspruch 33, worin die Erkrankung Herpes labialis ist.

35. Verfahren zur Herstellung eines festen arzneihaltigen Stifts gemäss mindestens einem der Ansprüche 1 bis 14, das die Inkorporierung von Acyclovir oder eines Esters oder eines Salzes davon und gegebenenfalls des anderen therapeutisch wirksamen Bestandteils in die geschmolzene Masse aus lipophilen Verdünnungsmitteln unter Rühren, und gegebenenfalls die Zugabe von einem oder mehreren Zusatzstoffen, Einfüllen der resultierenden geschmolzenen Masse in Formen für Lippenstifte und Abkühlen zur Verfestigung umfasst.

36. Verfahren zur Herstellung eines flüssigen arzneihaltigen Stifts gemäss mindestens einem der Ansprüche 15 bis 32, das die Auflösung der Formulierungsbestandteile in einem ausgewählten Lösungsmittel, dem gegebenenfalls ein Tensid zugegeben wurde, und anschliessendes Eingiessen der resultierenden Mischung in Roll-on-Stiftbehälter umfasst.

37. Topische Abgabevorrichtung, die eine Zusammensetzung gemäss mindestens einem der Ansprüche 1 bis 32 und einen Träger für feste Stifte oder einen Vorratsbehälter für Roll-on-Stifte umfasst.

## Revendications

1. Bâtonnet médicamenteux pour administration topique comprenant :
i) l'aciclovir ou un de ses dérivés pharmaceutiquement acceptables comme seul ingrédient actif, ou
ii) ledit aciclovir étant optionnellement associé à un ingrédient actif choisi dans le groupe constitué par le rétinol ou un de ses dérivés pharmaceutiquement acceptables, et/ou au moins un autre ingrédient actif antiviral,
ledit bâtonnet étant solide ou liquide (applicateur à bille).

2. Le bâtonnet médicamenteux solide selon la revendication 1, **caractérisé en ce qu'**il contient un véhicule comprenant au moins une substance grasse autre que les cires, au moins une cire naturelle, optionnellement au moins une substance paraffinnique (ou une cire synthétique), et optionnellement au moins un additif.

3. Le bâtonnet médicamenteux solide selon la revendication 2, **caractérisé en ce que** la substance grasse est choisie dans le groupe des glycérides, alcools gras, est d'alcools inférieurs et d'acides gras, la lanoline ou les alcools qui en dérivent, les stérols et les phospholipides.

4. Le bâtonnet médicamenteux solide selon la revendication 3, **caractérisé en ce que** les glycérides sont choisis parmi l'huile de ricin (optionnellement remplacée totalement ou partiellement par l'huile de ricin hydrogénée), l'huile de jojoba, l'huile de sésame, l'huile de maïs, l'huile de graine de coton, l'huile de palme, l'huile d'amande, l'huile de colza, l'huile d'olive, l'huile d'arachide, la stéarine, le beurre de cacao, les ersatz de beurre de cacao; les alcools gras sont choisis parmi les alcools cétylique, oléylique et stéarylique ; le stérol est le cholestérol ; les phospholipides sont des alpha-lécithines ; les esters gras sont des esters d'acides gras et d'alcools inférieurs de 1 à 6 atomes de carbone.

5. Le bâtonnet médicamenteux solide selon la revendication 1, dans lequel la cire est choisie parmi la cire blanche d'abeille, la cire de carnauba, le spermaceti, la cire de candelilla, la cire de baie de laurier, la cire de sucre de canne.

6. Le bâtonnet médicamenteux solide selon la revendication 1, **caractérisé en ce que** la substance paraffinique est choisie parmi les paraffines, la vaseline, et les huiles paraffiniques.

7. Le bâtonnet médicamenteux solide selon la revendication 2, **caractérisé en ce que** l'additif et choisi parmi les charges, les conservateurs et les parfums.

8. Le bâtonnet médicamenteux solide selon la revendication 7, **caractérisé en ce que** la charge est choisie parmi le talc, le carbonate de calcium, l'oxyde de zinc, le dioxyde de titane, les silicates de magnésium, l'aluminium micronisé, et les huiles siliconées ; et le conservateur est choisi parmi le butylhydroxy anisole, la butylhydroxy toluène, le 4-t-butyl-4'-méthoxy-dibenzoylméthane, les esters d'acide p-méthoxy-cinnamique et d'alcools en C₁-C₆ ; les esters de propyle, d'octyle ou de dodécyle et d'acide gallique, le tocophérol, le palmitate d'ascorbyle et le butyl-, le propyl- ou l'éthyl- paraben.

9. Le bâtonnet médicamenteux solide selon la revendication 2, **caractérisé en ce que** la quantité de substance grasse autre que le cires est de 20% à 90% en poids, la quantité de cire est de 1% à 40% en poids, la quantité de substance parafinnique est de 5% à 35% en poids, et la quantité d'additif, le cas échéant, est de 0,1% à 10% en poids, les pourcentages étant exprimés par rapport au poids total des ingrédients de la composition.

10. Le bâtonnet médicamenteux solide selon la revendication 2, **caractérisé en ce qu'**il comprend entre 60% et 70% en poids d'au moins une substance grasse autre que les cires et environ 10% o 30% en poids d'une cire, par rapport au poids total des ingrédients de la composition.

11. Le bâtonnet médicamenteux solide selon la revendication 2, **caractérisé en ce qu'**il comprend entre 30% et 80% en poids d'au moins une substance grasse autre que les cires, de 55% à 30% en poids d'au moins une cire, et de 10% à 30% en poids d'au moins une substance paraffinique.

12. Le bâtonnet médicamenteux solide selon la revendication 10 ou 11, **caractérisé en ce que** le bâtonnet solide comprend de 30% à 60% en poids d'huile de ricin, et optionnellement de 5% à 45% en poids d'une autre famille de substances grasses autres que les cires, choisie parmi la lanoline, un alcool gras, des glycérides semi-synthétiques, et des esters d'acides gras et d'alcools de C1-C6, les pourcentages étant exprimés en poids par rapport au poids total des ingrédients de la composition.

13. Le bâtonnet médicamenteux solide selon la revendication 2, **caractérisé en ce qu'**il comprend de 5% à 20% en poids d'une cire et de 30% à 80% en poids de triglycérides.

14. Le bâtonnet médicamenteux solide selon la revendication 13, **caractérisé en ce qu'**il comprend de 5% à 20% en poids d'une cire, de 50% à 80% en poids de triglycérides à chaîne moyenne et de 10% à 20% d'huile de ricin hydrogénée.

15. Le bâtonnet médicamenteux liquide selon la revendication 1, **caractérisé en ce qu'**il comprend de 20% à 30% en poids d'eau, de 0,05% à 1% en poids d'au moins un agent épaississant hydrophile, et au moins 10% à 50% en poids de monoéthyl éther de diéthylène glycol.

16. Le bâtonnet médicamenteux liquide selon la revendication 15, comprenant de 20% à 40% en poids de monoéthyl éther de diéthylène glycol et de 20% à 40% en poids de polyol en C₂-C₆.

17. Le bâtonnet médicamenteux liquide selon la revendication 15 ou 16, **caractérisé en ce que** l'agent épaississant est choisi parmi les gommes, les alginates, les polymères ou copolymères acryliques, les dérivés cellulosiques, les alcools polyvinyliques, et les esters de glycéryle, optionnellement au moins un plastifiant choisi parmi le citrate de triéthyle, l'acétate de dioctyle, des agents tensio-actifs non ioniques et des polyols miscibles à l'eau.

18. Le bâtonnet médicamenteux liquide selon l'une quelconque des revendications 15 à 17, **caractérisé en ce que** l'agent épaississant est le carboxypolyméthylène.

19. Le bâtonnet médicamenteux liquide selon la revendication 1, contenant un véhicule aqueux comprenant de l'eau, au moins un agent épaississant, combinés avec un co-solvant organique miscible à l'eau, **caractérisé en ce qu'**il contient au moins 50% en poids d'eau et moins de 30% en poids dudit co-solvant, et **en ce qu'**il est exempt de substance lipophile choisie parmi les cires, la lanoline, et les substances hydrophobes à base d'hydrocarbures.

20. Le bâtonnet médicamenteux liquide selon la revendication 19, contenant de 70% à 95% en poids d'eau, de 1% à 10% en poids d'agent épaississant hydrophobe, et moins de 30% en poids de co-solvant organique miscible à l'eau, par rapport au poids total de la composition.

21. Le bâtonnet médicamenteux liquide selon la revendication 19, dans lequel l'agent épaississant hydrophobe est le monostéarate de glycéryle, et le co-solvant organique miscible à l'eau est un polyol en C₂-C₆.

22. Le bâtonnet médicamenteux liquide selon la revendication 1, **caractérisé en ce qu'**il est une solution aqueuse contenant au moins un alcool en C₂-C₁₀, et optionnellement au moins un additif.

23. Le bâtonnet médicamenteux liquide selon la revendication 22, comprenant l'alcool choisi parmi l'alcool éthylique, l'alcool isopropylique et l'alcool benzylique.

24. Le bâtonnet médicamenteux liquide selon la revendication 22, dans lequel l'alcool est en une quantité de 10% à 40% en poids par rapport au poids total des ingrédients, et de 0,1% à 5% en poids d'additif.

25. Le bâtonnet médicamenteux liquide selon la revendication 1, **caractérisé en ce qu'**il comprend un véhicule aqueux comprenant des polyéthylène glycols avec un poids moléculaire moyen inférieur à 1000 ou des mono- ou di-esters avec des acides gras.

26. Le bâtonnet médicamenteux liquide selon la revendication 25, dans lequel les polyéthylène glycols (PEG) sont choisis parmi PEG 200, PEG 600 et leurs mélanges.

27. Le bâtonnet médicamenteux selon l'une quelconque des revendications 1 à 26, dans lequel le dérivé de rétinol est un ester choisi parmi l'acétate de rétinol et le palmitate de rétinol, ou l'acide rétinoïque.

28. Le bâtonnet médicamenteux selon l'une quelconque des revendications 1 à 27, comprenant du rétinol ou un de ses dérivés en quantité comprise entre 0,5% et 10% en poids par rapport au poids total des ingrédients du bâtonnet médicamenteux.

29. Le bâtonnet médicamenteux selon l'une quelconque des revendications 1 à 28, comprenant du rétinol ou un de ses esters en une quantité comprise entre 1% et 3% en poids par rapport au poids total des ingrédients du bâtonnet médicamenteux.

30. Le bâtonnet médicamenteux selon l'une quelconque des revendications 1 à 29, dans lequel ledit ingrédient actif antiviral est chois parmi la valaciclovir, le ganciclovir, le famciclovir, le penciclovir, la 9-(1,3-dihydroxy-2-propylméthyl)guanine, les dérivés phosphatés d'aciclovir, les dérivés de phosphotriesters ou d'esters de valine, la vidabarine, la cytarabine, l'acide phosphonoacétique, l'acide phosphonoformique, l'idoxuridine, la trifluridine, l'edoxudine, le brovavir, la flacitabine et le rociclovir.

31. Le bâtonnet médicamenteux selon l'une quelconque des revendications 1 à 30, **caractérisé en ce que** la quantité d'aciclovir ou de dérivé d'aciclovir qui y est contenue est de 0,5% à 10% en poids par rapport au poids total des ingrédients.

32. Le bâtonnet médicamenteux selon la revendication 31, **caractérisé en ce que** ladite quantité est entre 2% et 5% en poids.

33. L'utilisation de l'aciclovir ou d'un de ses dérivés pharmaceutiquement acceptables pour la fabrication d'une composition pharmaceutique pour le traitement topique d'une maladie virale, **caractérisée en ce que** ladite composition est un bâtonnet médicamenteux tel que défini dans l'une quelconque des revendications 1 à 32.

34. L'utilisation selon la revendication 32, dans laquelle ladite maladie virale est l'herpès labial.

35. Procédé de préparation d'un bâtonnet médicamenteux solide selon l'une quelconque des revendications 1 à 14, comprenant l'incorporation sous agitation d'aciclovir ou d'un de ses sels ou esters, et optionnellement les autres ingrédients thérapeutiquement actifs, dans la masse fondue des excipients lipophiles, l'addition optionnelle d'un ou plusieurs additifs, le chargement de la masse fondue résultante dans un moules pour bâtonnets à lèvres, et le refroidissement jusqu'à solidification.

36. Procédé de préparation d'un bâtonnet médicamenteux liquide selon l'une quelconque des revendications 15 à 32, qui comprend la dissolution des ingrédients de la formulation dans un solvant choisi, auquel un agent tensio-actif est optionnellement ajouté, puis le versement du mélange résultant dans des réservoirs à applicateurs à bille.

37. Dispositif d'administration topique comprenant une composition telle que revendiquée dans l'une quelconque des revendications 1 à 32, et un support pour bâtonnets solides ou un réservoir pour applicateur à bille.
